# FASCICULE DE BREVET EUROPEEN

(11) **EP 1 303 253 B1**
(45) Date de publication et mention de la délivrance du brevet: **15.09.2004**
(21) Numéro de dépôt: 01945388.5
(22) Date de dépôt: 08.06.2001
(51) Int. Cl.: A61K 7/48, A61K 7/06

(54) **COMPOSITION, NOTAMMENT COSMETIQUE, COMPRENANT LA DHEA ET/OU UN PRECURSEUR OU DERIVE DE CELLE-CI, EN ASSOCIATION AVEC AU MOINS UN AGENT ANTI-GLYCATION**
ZUSAMMENSETZUNG, INSBESONDERE FÜR KOSMETIKA, ENTHALTEND DHEA UND/ODER EINEN VORLÄUFER ODER DERIVAT DAVON IN KOMBINATION MIT MINDESTENS EINEM GLYKOSYLIERUNG-INHIBITOR
COMPOSITION, IN PARTICULAR COSMETIC, COMPRISING DHEA AND/OR A PRECURSOR OR DERIVATIVE THEREOF, COMBINED WITH AT LEAST A GLYCATION INHIBITING AGENT

(30) Priorité: 13.07.2000 FR 0009232
(43) Date de publication de la demande: 23.04.2003
(73) Titulaire: L'OREAL, 75008 Paris (FR)
(72) Inventeur: COURCHAY, Guy, F-94370 Sucy-en-Brie (FR); ROGUET, Roland, F-75020 Paris (FR)
(74) Mandataire: Renard, Emmanuelle
(86) Numéro de dépôt international: PCT/FR2001/001790
(87) Numéro de publication internationale: WO 2002/005777

(56) Documents cités:
- EP-A- 0 908 183
- EP-A- 1 068 864
- FR-A- 2 776 188
- FR-A- 2 777 183
- FR-A- 2 795 643
- US-A- 5 989 568
- DATABASE WPI Section Ch, Week 199810 Derwent Publications Ltd., London, GB; Class B04, AN 1998-105073 XP002168799 & JP 09 328410 A (MITSUBA BOEKI KK), 22 décembre 1997 (1997-12-22)

## Description

L'invention se rapporte à une composition comprenant, dans un milieu physiologiquement acceptable, la DHEA et/ou un précurseur ou dérivé chimique ou biologique de celle-ci, en association avec au moins un agent anti-glycation, ainsi qu'à son utilisation pour prévenir ou traiter les signes de vieillissement de la peau.

La glycation est un processus non enzymatique faisant intervenir un ose (glucose ou ribose) qui réagit selon la réaction de Maillard avec un groupement aminé d'un résidu d'acide aminé (comme par exemple la lysine), particulièrement un résidu d'acide aminé d'une protéine, pour former une base de Schiff. Celle-ci, après un réarrangement moléculaire dit d'Amadori, peut conduire, par une succession de réactions, à un pontage, particulièrement intramoléculaire comme par exemple de type pentosidine. Ce phénomène se caractérise par l'apparition de produits de glycation dont la teneur augmente de façon régulière en fonction de l'âge. Les produits de glycation sont par exemple la pyrraline, la carboxyméthyl-lysine, la pentosidine, la crossline, la N^{ε}(2-carboxyéthyl)-lysine (CEL), la glyoxal-lysine dimer (GOLD), la méthylglyoxal-lysine dimer (MOLD), la 3DG-ARG imidazolone, les versperlysines A, B, C, la thréosidine ou encore les produits finaux de glycosylation avancée (ou AGEs).

La glycation des protéines est donc un phénomène universel, bien connu au niveau de la peau, particulièrement au niveau de sa composante dermique, et principalement au niveau des fibres de collagène. La glycation du collagène augmente en effet de façon régulière avec l'âge, entraînant une augmentation régulière de la teneur de la peau en produits de glycation.

Ainsi, au cours du vieillissement de la peau, les propriétés physico-chimiques du collagène se modifient et ce dernier devient plus difficilement soluble et plus difficilement dégradable. Il s'ensuit une rigidification des tissus conduisant essentiellement à une perte de tonicité de la peau.

Outre ses effets sur le vieillissement de la peau, la glycation intervient dans l'aspect "peau d'orange" caractéristique de la cellulite. Dans la cellulite, en effet, la glycation du collagène constituant la plus grande part des travées conjonctives entraîne une rigidification des tissus qui emprisonnent alors les globules graisseux. La peau présente ainsi une succession de bosses formées d'amas graisseux et de creux formés des travées conjonctives rigidifiées, caractéristiques de l'aspect "peau d'orange".

On comprend donc l'importance qui existe à disposer de produits qui diminuent voire inhibent le phénomène de glycation des protéines.

On connaît différents produits capables d'inhiber cette réaction de glycation : l'aminoguanidine (qui est l'inhibiteur le plus connu), la taurine, certaines vitamines (B1, B6), les dérivés thiazolium, les extraits végétaux de la famille des Ericaceae (tels que les extraits de myrtille), et les hydroxystilbènes et leurs dérivés, tels que le resvératrol et le 3,3',5,5'-tétrahydroxystilbène.

Toutefois, ces composés ne permettent pas toujours de lutter de façon suffisamment efficace contre les signes du vieillissement cutané.

Or, il est apparu à la Demanderesse que la DHEA et/ou ses précurseurs ou dérivés chimiques ou biologiques pouvaient permettre d'améliorer l'efficacité anti-âge des compositions comprenant des agents anti-glycation.

Le document brevet FR-A-2 776 188 décrit une composition comprenant un agent anti-glycation (oléamide de glycylglycine), auquel on peut adjoindre de la DHEA.

La DHEA, ou déhydroépiandrostérone, est un stéroïde naturel produit essentiellement par les glandes corticosurrénales. Elle est connue pour sa capacité à promouvoir la kératinisation de l'épiderme (JP-07 196 467), ou encore dans le traitement des peaux sèches, en raison de son aptitude à augmenter la production endogène et la sécrétion de sébum et à renforcer l'effet barrière de la peau (US-4,496,556). Il a également été décrit dans le brevet US-5,843,932 l'utilisation de la DHEA pour remédier à l'atrophie du derme par inhibition de la perte de collagène et de tissu conjonctif. Il a en outre été décrit dans le brevet US-5,736,537 l'utilisation par voie orale d'esters de DHEA, en particulier du salicylate de DHEA, pour réguler l'atrophie de la peau due à un amincissement ou une dégradation générale du derme. Il a enfin été proposé d'utiliser le sulfate de DHEA pour traiter différents signes du vieillissement tels que les rides, la perte d'éclat de la peau et le relâchement cutané (EP-0 723 775).

Toutefois, à la connaissance de la Demanderesse, il n'a encore jamais été suggéré d'associer un des précurseurs chimiques de la DHEA du type sapogénine à un composé anti-glycation.

Outre ses applications dans la prévention ou le traitement du vieillissement cutané, une telle association peut se révéler utile dans la prévention et/ou le traitement de l'aspect "peau d'orange". En effet, sans vouloir être liée par cette théorie, la Demanderesse pense que l'agent anti-glycation pourrait renforcer l'effet de la DHEA dans des compositions destinées au traitement de la cellulite ou de la peau d'orange, dans lesquelles la DHEA peut elle-même être utilisée en raison de ses propriétés de blocage de la différenciation des fibroblastes en adipocytes (Shantz et al., Mechanism of Inhibition of growth of 3T3-L1 fibrobiasts and their differentiation to adipocytes by DHEA and related steroids : role of glucose-6-phosphate dehydrogenase, Proc. Natl. Acad. Sci. USA, 86(10):3852-6, 1989).

L'invention a donc pour objet une composition comprenant, dans un milieu physiologiquement acceptable, une sapogénine ou un extrait naturel en contenant en association avec au moins un agent anti-glycation.

La DHEA a la formule (I) suivante :

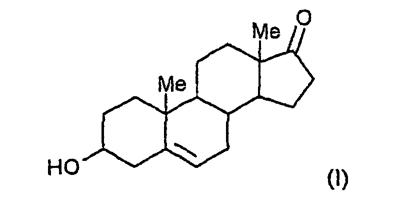

Elle est par exemple disponible auprès de la société AKZO NOBEL.

Par précurseurs de la DHEA, on entend ses précurseurs chimiques qui peuvent se transformer en DHEA par réaction chimique exogène. Des exemples de précurseurs chimiques sont les sapogénines, tels que la diosgénine (ou spirost-5-èn-3-beta-ol), l'hécogénine, l'acétate d'hécogénine, le smilagénine et la sarsapogénine, ainsi que les extraits naturels en contenant, en particulier le fenugrec et les extraits de Dioscorées telles que la racine d'igname sauvage ou Wild Yam, sans que cette liste soit limitative.

Les précurseurs et dérivés biologiques et chimiques de DHEA seront désignés ci-dessous par "analogues de DHEA".

De son côté, l'agent anti-glycation est un agent prévenant et/ou diminuant la glycation des protéines de la peau, en particulier des protéines du derme telles que le collagène.

Des exemples d'agents anti-glycation sont les extraits végétaux de la famille des Ericaceae, tels que les extraits de myrtille, l'ergothionéine et ses dérivés, et les hydroxystilbènes et leurs dérivés, tels que le resvératrol et le 3,3',5,5'-tétrahydroxystilbène. Ces agents anti-glycation sont décrits dans les demandes FR 99/16166, FR 00/08158, FR 99/09267 et FR 99/16168, respectivement. Le resvératrol est particulièrement préféré pour une utilisation dans cette invention.

La composition selon l'invention peut être utilisée à des fins cosmétiques, soit pour prévenir ou traiter les signes du vieillissement cutané, en particulier pour prévenir ou traiter la perte de tonicité de la peau, soit pour prévenir ou traiter l'aspect peau d'orange.

Cette composition peut être destinée à une application topique sur la peau. Dans ce cas, la DHEA et/ou ses analogues peuvent être présents en une quantité allant de 10⁻⁶ à 10% en poids, et de préférence de 0,1 à 5% en poids, par rapport au poids total de la composition. Mieux, la DHEA et/ou ses analogues peuvent être présents en une quantité d'environ 1% en poids, par rapport au poids total de la composition. L'agent anti-glycation peut représenter de 0,001 à 10% du poids total de la composition.

La composition selon l'invention peut alors se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, en particulier sous forme de stick. Elle peut être utilisée comme produit de soin et/ou comme produit de maquillage pour la peau.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées de l'association d'actifs selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Pour une utilisation en vue de prévenir ou traiter la perte de tonicité de la peau, la composition selon l'invention peut comprendre d'autres actifs anti-âge, en particulier des actifs anti-rides, dépigmentants, drainants, anti-radicalaires et/ou immunoprotecteurs et/ou à des agents stimulant le métabolisme cellulaire (notamment la synthèse des protéines) et/ou la prolifération cellulaire.

Pour une utilisation en vue de prévenir ou traiter l'aspect peau d'orange, la composition selon l'invention peut comprendre l'ergothionéine peut être associée à des actifs raffermissants, drainants, astringents, lipolytiques, tenseurs et/ou desquamants.

En cas d'incompatibilité, certains au moins des actifs mentionnés ci-dessus peuvent être incorporés dans des sphérules, notamment des vésicules ioniques ou non-ioniques et/ou des nanoparticules (nanocapsules et/ou nanosphères), de manière à ce que les actifs incompatibles entre eux soient isolés les uns des autres dans la composition.

Selon une autre possibilité, la composition selon l'invention peut être destinée à une administration par voie orale.

La présente invention concerne donc également un procédé cosmétique de prévention ou de traitement des signes du vieillissement cutané, comprenant l'administration par voie orale ou topique d'une composition comprenant la DHEA et/ou un précurseur ou dérivé chimique ou biologique de celle-ci, en association avec au moins un agent anti-glycation.

Dans le cas où la composition est administrée par voie orale, la DHEA et/ou ses analogues peut être administrée à raison de 1 à 100 mg/jour, de préférence de 25 à 75 mg/jour et l'agent anti-glycation peut être administré à raison de 1 à 100 mg/jour, de préférence de 25 à 75 mg/jour.

Cette composition peut se présenter sous toute forme galénique convenant à ce mode d'administration, par exemple sous forme de comprimés sécables ou non, de granules, de capsules, de gélules, de solutés, de suspensions ou de solutions comprenant un excipient approprié.

L'invention sera mieux comprise, et ses avantages ressortiront mieux, à la lumière des exemples suivants, qui sont donnés à titre illustratif, et sans limitation. Les quantités indiquées dans ces exemples sont données en pourcentage pondéral, sauf indication contraire.

### EXEMPLES

### Exemple 1 - Composition anti-âge pour administration orale

On prépare, de manière classique pour l'homme du métier, des capsules molles ayant la composition suivante :

| | |
|---|---|
| Huile de soja hydrogénée | 40 mg |
| Huile de blé | 95 mg |
| Lécithine de soja | 20 mg |
| Tocophérols naturels | 5 mg |
| Acide ascorbique | 30 mg |
| Resvératrol | 50 mg |
| DHEA | 50 mg |

### Exemple 2 -Composition anti-âge pour application topique

On prépare, de manière classique pour l'homme du métier, une crème de soin (émulsion huile dans eau) ayant la composition suivante :

| | |
|---|---|
| Resvératrol | 1 % |
| DHEA | 0,1 % |
| Stearate de glycérol | 2 % |
| Polysorbate 60 (Tween 60 ® vendu par la société ICI) | 1 % |
| Acide stearique | 1,4 % |
| Triethanolamine | 0,7 % |
| Carbomer | 0,4 % |
| Fraction liquide de beurre de karité | 12 % |
| Perhydrosqualène | 12 % |
| Parfum | 0,5 % |
| Conservateur | QS |
| Eau QSP | 100 % |

### Exemple 3 : Crème anti-peau d'orange

| | |
|---|---|
| Ergothionéine | 1 % |
| Sodium diméthicone copolyol acétyl méthyltaurate (Pecosil DCT de la société PHOENIX) | 5,4 % |
| Huile minérale | 19 % |
| Glycérine | 5 % |
| Conservateurs | 0,25% |
| DHEA | 1 % |
| Gomme de xanthane | 0,25% |
| Sel d'ammonium de l'acide acrylamidométhylpropane sulfonique | 1,2 % |
| Mélange de cyclopentasiloxane et diméthiconol | 5 % |
| Extrait de *Gingko biloba* dans le propylène glycol | 5 % |
| Eau qsp. | 100 % |

## Revendications

1. Composition comprenant, dans un milieu physiologiquement acceptable, une sapogénine ou un extrait naturel en contenant, en association avec au moins un agent anti-glycation.

2. Composition selon la revendication 1, **caractérisée en ce que** ladite sapogénine est choisie parmi la diosgénine, l'hécogénine, le smilagénine et la sarsapogénine. ,

3. Composition selon la revendication 1, **caractérisée en ce que** ledit extrait naturel est choisi parmi le fenugrec et les extraits de Dioscorées.

4. Composition selon la revendication 3, **caractérisée en ce que** ledit extrait de Dioscorée est un extrait de racine d'igname sauvage.

5. Composition selon l'une quelconque des revendications précédentes, **caractérisée en ce que** l'agent anti-glycation est un agent prévenant et/ou diminuant la glycation des protéines de la peau.

6. Composition selon la revendication 5, **caractérisée en ce que** les protéines de la peau sont les protéines du derme.

7. Composition selon l'une quelconque des revendications précédentes, **caractérisée en ce que** l'agent anti-glycation est le resvératrol.

8. Composition selon l'une quelconque des revendications précédentes, **caractérisée en ce qu'**elle est destinée à une application topique sur la peau.

9. Composition selon la revendication 8, **caractérisée en ce que** la sapogénine est présente en une quantité allant de 10⁻⁶ à 10% en poids, par rapport au poids total de la composition.

10. Composition selon la revendication 9, **caractérisée en ce que** la sapogénine est présente en une quantité allant de 0,1 à 5% en poids, par rapport au poids total de la composition.

11. Composition selon la revendication 10, **caractérisée en ce que** la sapogénine est présente en une quantité d'environ 1% en poids, par rapport au poids total de la composition.

12. Composition selon l'une quelconque des revendications 8 à 11, **caractérisée en ce que** l'agent anti-glycation représente de 0,001 à 10% du poids total de la composition.

13. Composition selon l'une quelconque des revendications 1 à 7, **caractérisée en ce qu'**elle est destinée à une administration par voie orale.

14. Procédé cosmétique de prévention ou de traitement des signes du vieillissement cutané, comprenant l'administration par voie orale ou topique d'une composition comprenant une sapogénine ou un extrait naturel en contenant, en association avec au moins un agent anti-glycation.

15. Procédé selon la revendication 14, **caractérisé en ce que** la sapogénine est administrée par voie orale à raison de 1 à 100 mg/jour, de préférence de 25 à 75 ma/jour.

16. Procédé selon la revendication 14, **caractérisé en ce que** l'agent anti-glycation est administré à raison de 1 à 100 mg/jour, de préférence de 25 à 75 mg/jour.

17. Utilisation cosmétique de la composition selon l'une quelconque des revendications 1 à 13, pour prévenir ou traiter les signes du vieillissement cutané.

18. Utilisation cosmétique de la composition selon l'une quelconque des revendications 1 à 13, pour prévenir ou traiter la perte de tonicité de la peau.

19. Utilisation cosmétique de la composition selon l'une quelconque des revendications 1 à 13, pour prévenir ou traiter l'aspect peau d'orange.

## Patentansprüche

1. Zusammensetzung, die in einem physiologisch akzeptablen Medium ein Sapogenin oder einen ein Sapogenin enthaltenden natürlichen Extrakt in Kombination mit mindestens einem Wirkstoff gegen Glykation enthält.

2. Zusammensetzung nach Anspruch 1, **dadurch gekennzeichnet, dass** das Sapogenin unter Diosgenin, Hecogenin, Smilagenin und Sarsapogenin ausgewählt ist.

3. Zusammensetzung nach Anspruch 1, **dadurch gekennzeichnet; dass** der natürliche Extrakt unter Bockshornklee und den Extrakten von Dioscoreaceae ausgewählt ist.

4. Zusammensetzung nach Anspruch 3, **dadurch gekennzeichnet, dass** der Dioscoreaceae-Extrakt ein Extrakt der Wurzel von Wildem Yams ist.

5. Zusammensetzung nach einem der vorhergehenden Ansprüche, **dadurch gekennzeichnet, dass** es sich bei dem Wirkstoff gegen Glykation um einen Wirkstoff handelt, der der Glykation der Proteine der Haut vorbeugt und/oder diese vermindert.

6. Zusammensetzung nach Anspruch 5, **dadurch gekennzeichnet, dass** die Proteine der Haut Proteine der Dermis sind.

7. Zusammensetzung nach einem der vorhergehenden Ansprüche, **dadurch gekennzeichnet, dass** der Wirkstoff gegen Glykation Resveratrol ist.

8. Zusammensetzung nach einem der vorhergehenden Ansprüche, **dadurch gekennzeichnet, dass** sie für eine topische Anwendung auf die Haut vorgesehen ist.

9. Zusammensetzung nach Anspruch 8, **dadurch gekennzeichnet, dass** das Sapogenin in einem Mengenanteil von 10⁻⁶ bis 10 Gew.-%, bezogen auf das Gesamtgewicht der Zusammensetzung, vorliegt.

10. Zusammensetzung nach Anspruch 9, **dadurch gekennzeichnet, dass** das Sapogenin in einem Mengenanteil von 0,1 bis 5 Gew.-%, bezogen auf das Gesamtgewicht der Zusammensetzung, vorliegt.

11. Zusammensetzung nach Anspruch 10, **dadurch gekennzeichnet, dass** das Sapogenin in einem Mengenanteil von etwa 1 Gew.-%, bezogen auf das Gesamtgewicht der Zusammensetzung, vorliegt.

12. Zusammensetzung nach einem der Ansprüche 8 bis 11, **dadurch gekennzeichnet, dass** der Wirkstoff gegen Glykation 0,001 bis 10 % des Gesamtgewichts der Zusammensetzung ausmacht.

13. Zusammensetzung nach einem der Ansprüche 1 bis 7, **dadurch gekennzeichnet, dass** sie für eine Verabreichung auf oralem Weg vorgesehen ist.

14. Kosmetisches Verfahren zur Vorbeugung oder zur Behandlung der Zeichen der Hautalterung, das die Verabreichung einer Zusammensetzung, die ein Sapogenin oder einen ein Sapogenin enthaltenden natürlichen Extrakt in Kombination mit mindestens einem Wirkstoff gegen Glykation enthält, auf oralem oder topischem Weg umfasst.

15. Verfahren nach Anspruch 14, **dadurch gekennzeichnet, dass** das Sapogenin auf oralem Weg in einer Menge von 1 bis 100 mg/Tag und vorzugsweise von 25 bis 75 mg/Tag verabreicht wird.

16. Verfahren nach Anspruch 14, **dadurch gekennzeichnet, dass** der Wirkstoff gegen Glykation in einer Menge von 1 bis 100 mg/Tag und vorzugsweise von 25 bis 75 mg/Tag verabreicht wird.

17. Kosmetische Verwendung der Zusammensetzung nach einem der Ansprüche 1 bis 13 zur Vorbeugung oder zur Behandlung der Zeichen der Hautalterung.

18. Kosmetische Verwendung der Zusammensetzung nach einem der Ansprüche 1 bis 13 zur Vorbeugung oder zur Behandlung des Spannkraftverlusts der Haut.

19. Kosmetische Verwendung der Zusammensetzung nach einem der Ansprüche 1 bis 13 zur Vorbeugung oder zur Behandlung der Erscheinung der Orangenhaut.

## Claims

1. Composition comprising, in a physiologically acceptable medium, a sapogenin or a natural extract comprising it, in combination with at least one antiglycation agent.

2. Composition according to Claim 1, **characterized in that** said sapogenin is chosen from diosgenin, hecogenin, smilagenin and sarsapogenin.

3. Composition according to Claim 1, **characterized in that** said natural extract is chosen from fenugreek and extracts of Dioscoreae.

4. Composition according to Claim 3, **characterized in that** said extract of Dioscorea is a wild yam root extract.

5. Composition according to any one of the preceding claims, **characterized in that** the antiglycation agent is an agent which prevents and/or reduces glycation of the proteins of the skin.

6. Composition according to Claim 5, **characterized in that** the proteins of the skin are the proteins of the dermis.

7. Composition according to any one of the preceding claims, **characterized in that** the antiglycation agent is resveratrol.

8. Composition according to any one of the preceding claims, **characterized in that** it is intended for topical application to the skin.

9. Composition according to Claim 8, **characterized in that** the sapogenin is present in an amount ranging from 10⁻⁶ to 10% by weight, with respect to the total weight of the composition.

10. Composition according to Claim 9, **characterized in that** the sapogenin is present in an amount ranging from 0.1 to 5% by weight, with respect to the total weight of the composition.

11. Composition according to Claim 10, **characterized in that** the sapogenin is present in an amount of approximately 1% by weight, with respect to the total weight of the composition.

12. Composition according to any one of Claims 8 to 11, **characterized in that** the antiglycation agent represents from 0.001 to 10% of the total weight of the composition.

13. Composition according to any one of Claims 1 to 7, **characterized in that** it is intended for administration by the oral route.

14. Cosmetic process for preventing or treating signs of cutaneous aging, comprising the administration, by the oral or topical route, of a composition comprising a sapogenin or a natural extract comprising it, in combination with at least one antiglycation agent.

15. Process according to Claim 14, **characterized in that** the sapogenin is administered by the oral route in a proportion of 1 to 100 mg/day, preferably of 25 to 75 mg/day.

16. Process according to Claim 14, **characterized in that** the antiglycation agent is administered in a proportion of 1 to 100 mg/day, preferably of 25 to 75 mg/day.

17. Cosmetic use of the composition according to any one of Claims 1 to 13, for preventing or treating signs of cutaneous aging.

18. Cosmetic use of the composition according to any one of Claims 1 to 13, for preventing or treating loss of tonicity of the skin.

19. Cosmetic use of the composition according to any one of Claims 1 to 13, for preventing or treating the orange peel appearance.
